# EUROPEAN PATENT SPECIFICATION

(11) **EP 3 970 771 B1**
(45) Date of publication and mention of the grant of the patent: **16.07.2025**
(21) Application number: 20806418.8
(22) Date of filing: 14.05.2020
(51) Int. Cl.: A61M 5/42, A61B 5/15, A61M 5/32

(54) **PUNCTURE-ASSISTANCE TOOL**
PUNKTIONSHILFSINSTRUMENT
OUTIL D'AIDE A LA PONCTION

(30) Priority: 14.05.2019 JP 2019091580
(43) Date of publication of application: 23.03.2022
(73) Proprietor: The School Corporation Kansai University, Suita-shi, Osaka 564-8680 (JP); Aiki Riotech Corporation, Inazawa-shi, Aichi 492-8162 (JP)
(72) Inventor: AOYAGI, Seiji, Suita-shi, Osaka 564-8680 (JP); MATSUMOTO, Hajime, Inazawa-shi, Aichi 492-8162 (JP)
(74) Representative: Vidon Brevets & Stratégie
(86) International application number: PCT/JP2020/019270
(87) International publication number: WO 2020/230849

(56) References cited:
- JP-A- 2003 516 205
- JP-A- 2010 005 398
- JP-A- 2015 532 180
- JP-A- S60 226 317
- JP-U- S6 091 804
- US-A1- 2004 181 203
- US-A1- 2005 137 531

## Description

### Technical Field

The present invention relates to, for example, a puncture assisting tool that is used to insert a needle into the skin of a living body.

### Background Art

In the collection of blood or the like of a living body, a puncture assisting tool is used to insert a needle into the skin of the living body. For example, Patent Literature 1 discloses a puncture instrument that includes a suction cup, a gripping part, a cavity, a nonreturn valve, and an injection needle. In the technique disclosed in Patent Literature 1, by lifting the gripping part up, a negative pressure is generated inside the cavity so that the suction cup suction-attracts the skin. Then, by releasing the gripping part, the injection needle is inserted into the skin.

The American patent application US 2004/181203 and US 2005/137531 disclose a device which either suctions the skin or adheres to and stretches the skin, but do not disclose nor teach a combination of both functions. Citation List

### [Patent Literature]

[Patent Literature 1]
Japanese Patent Application Publication Tokukai No. 2009-112416

### Summary of Invention

### Technical Problem

Unfortunately, the technique of Patent Literature 1 causes the skin to be depressed through the application of pressure by the injection needle during the insertion of the injection needle. The depression in the skin stimulates a pain sensory nerve of a living body and thus causes pain to the living body.

An aspect of the present invention has an object to achieve a puncture assisting tool that makes it possible to prevent skin from being depressed during insertion of a needle into the skin.

### Solution to Problem

In order to attain the object, a puncture assisting tool in accordance with an aspect of the present disclosure includes: a first fixing part that is fixed to a first region of skin; and a second fixing part that is fixed to a second region of the skin; the first fixing part being movable relative to the second fixing part so as to pull the skin between the first fixing part and the second fixing part.

### Advantageous Effects of Invention

An aspect of the present invention makes it possible to prevent skin from being depressed during insertion of a needle into the skin.

### Brief Description of Drawings

Fig. 1 is a perspective view of a puncture assisting tool in accordance with Embodiment 1 of the present invention.
Fig. 2 illustrates the puncture assisting tool as viewed from an upward direction in Fig. 1.
Fig. 3 is a cross-sectional view taken along the line A-A illustrated in Fig. 2.
Fig. 4 is a view for describing an example of a puncture method in which the puncture assisting tool is used.
Fig. 5 is a view for describing an example of a puncture method in which the puncture assisting tool is used.
Fig. 6 is a view for describing an example of a puncture method in which the puncture assisting tool is used.
Fig. 7 is a view for describing an example of a puncture method in which the puncture assisting tool is used.
Fig. 8 is a perspective view of a puncture assisting tool in accordance with Embodiment 2 of the present invention.
Fig. 9 is a side view of the puncture assisting tool.
Fig. 10 is a perspective view of a puncture assisting tool in accordance with Embodiment 3 of the present invention.
(a) of Fig. 11 is a side view of the puncture assisting tool. (b) of Fig. 11 is an enlarged view of a region surrounded with a frame in (a) of Fig. 11.
Fig. 12 is a perspective view of a puncture assisting tool in accordance with Embodiment 4 of the present invention.
Fig. 13 is a cross-sectional view taken along the line B-B illustrated in Fig. 12.
(a) to (c) of Fig. 14 are views each for describing an example of a puncture method in which the puncture assisting tool is used.
Fig. 15 is a top view of a puncture assisting tool in accordance with Embodiment 5 of the present invention.
Fig. 16 is a cross-sectional view taken along the line C-C illustrated in Fig. 15.
(a) to (c) of Fig. 17 are views each for describing an example of a puncture method in which the puncture assisting tool is used.
Fig. 18 is a perspective view of a puncture assisting tool in accordance with Embodiment 6 of the present invention.
Fig. 19 is a perspective view of a puncture assisting tool in accordance with Embodiment 7 of the present invention.
Fig. 20 illustrates the puncture assisting tool as viewed from an upward direction in Fig. 19.
Fig. 21 is a cross-sectional view taken along the line D-D of Fig. 20 and illustrating a state in which pressure inside a frame of an attachment part is reduced during puncture in which the puncture assisting tool is used.

### Description of Embodiments

### Embodiment 1

The following description will discuss an embodiment of the present invention in detail.

### (Configuration of puncture assisting tool 1)

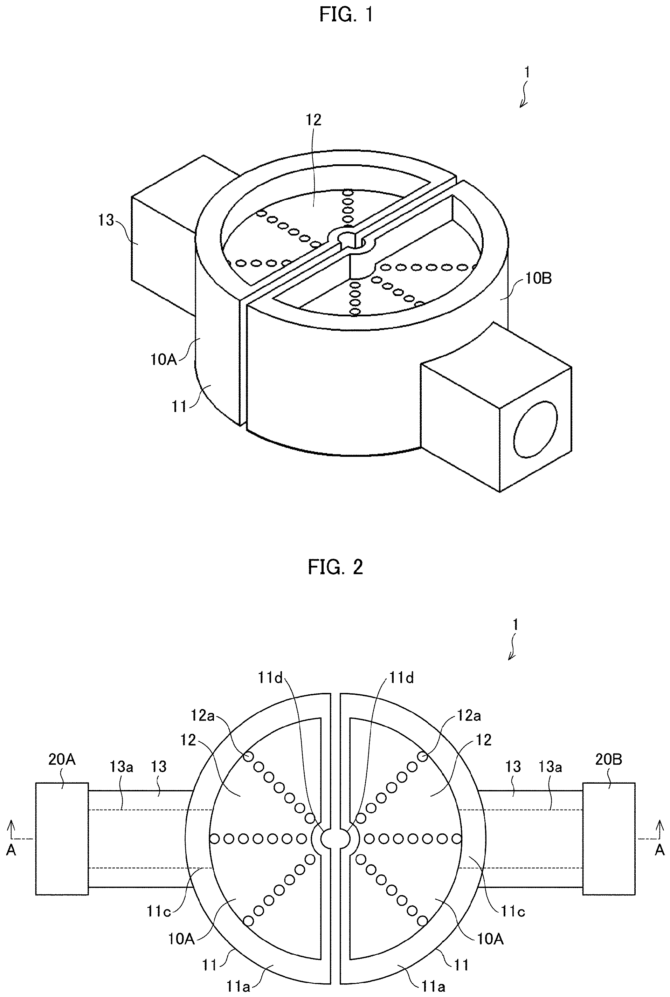

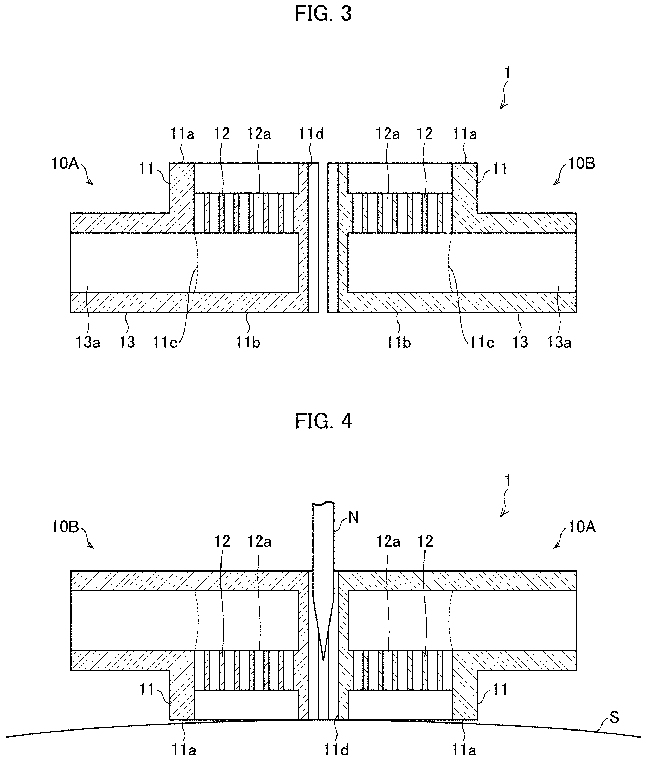

Fig. 1 is a perspective view of the puncture assisting tool 1. Fig. 2 illustrates the puncture assisting tool 1 as viewed from an upward direction in Fig. 1. Fig. 3 is a cross-sectional view taken along the line A-A illustrated in Fig. 2.

As illustrated in Figs. 1 to 3, the puncture assisting tool 1 includes a first fixing part 10A, a second fixing part 10B, a pump 20A, and a pump 20B. Note that for simplification, Figs. 1 and 3 do not illustrate the pump 20A (suction device) and the pump 20B (suction device).

The first fixing part 10A and the second fixing part 10B have shapes that are symmetrical in the transverse direction in Figs. 2 and 3. Thus, only the first fixing part 10A will be described herein. The first fixing part 10A includes a frame 11, a support 12, and an air discharge part 13.

The frame 11 has a semicylindrical shape obtained by cutting a cylinder along a plane containing a central axis. The frame 11 includes: a first end 11a, which is one end of a semicylinder and is an end on a side that contacts the skin of a living body; and a second end 11b, which is another end of the semicylinder. The first end 11a has a semicircular shape and has an opening on the inside, and the second end 11b is made of a semicircular flat plate. Thus, the frame 11 has a shape in which a face thereof on the side that contacts the skin of a living body is open.

The frame 11 includes an opening 11c formed on a curved side surface of the semicylinder and in a region where the air discharge part 13 (described later) is installed. Furthermore, the frame 11 includes a groove 11d formed on a flat side surface of the semicylinder so as to be recessed inward.

The support 12, in an inner part of the frame 11, is formed between the first end 11a and the second end 11b in the axial direction (vertical direction in Fig. 3) of the semicylindrical shape of the frame 11. The support 12 is made of a semicircular flat plate having a radius of the same length as an inner diameter of the frame 11. As illustrated in Figs. 1 to 3, the support 12 has a plurality of air intake holes 12a formed therein that penetrate in the axial direction of the semicylindrical shape of the frame 11. Note that although Figs. 1 to 3 differ in number of the air intake holes 12a formed in the support 12, the number (density) of the air intake holes 12a formed in the support 12 is not particularly limited, and can be set as appropriate, provided that the strength of the support 12 is not greatly decreased.

The air discharge part 13 connects a space in the inner part of the frame 11 and the pump 20A to each other. The air discharge part 13 is provided on the side surface of the frame 11. The air discharge part 13 has a cylindrical hole 13a formed therein. The hole 13a is connected at one end thereof to the opening 11c, which is provided in the frame 11. The hole 13a is connected at another end thereof to the pump 20A, as illustrated in Fig. 2.

The pump 20A is connected to the air discharge part 13 of the frame 11 of the first fixing part 10A. The pump 20A reduces pressure inside the frame 11 of the first fixing part 10A by sucking, via the air discharge part 13, air inside the frame 11 of the first fixing part 10A.

The pump 20B is connected to the air discharge part 13 of the frame 11 of the second fixing part 10B. The pump 20A reduces pressure inside the frame 11 of the second fixing part 10B by sucking, via the air discharge part 13, air inside the frame 11 of the second fixing part 10B.

### (Examples of puncture method in which puncture assisting tool 1 is used)

The following description will discuss an example of a puncture method in which the puncture assisting tool 1 is used. Figs. 4 to 7 are views each for describing an example of the puncture method in which the puncture assisting tool 1 is used.

According to the puncture method in which the puncture assisting tool 1 is used, first, the puncture assisting tool 1 (more specifically, the first end 11a of the frame 11 of the first fixing part 10A and the first end 11a of the frame 11 of the second fixing part 10B) is brought into contact with skin S of a living body (see Fig. 4). In this state, a needle N placed at a tip of a syringe (not illustrated) is located between the groove 11d formed in the frame 11 of the first fixing part 10A and the groove 11d formed in the frame 11 of the second fixing part 10B, and the needle N is not inserted into the skin S of the living body. Furthermore, the first fixing part 10A and the second fixing part 10B are in contact with each other in this state.

Next, the pump 20A and the pump 20B are driven so that the pressure inside the frame 11 of the first fixing part 10A and the pressure inside the frame 11 of the second fixing part 10B are reduced. Note that since the support 12 has the plurality of air intake holes 12a formed therein, it is possible to reduce the pressure in the whole inner part of the frame 11.

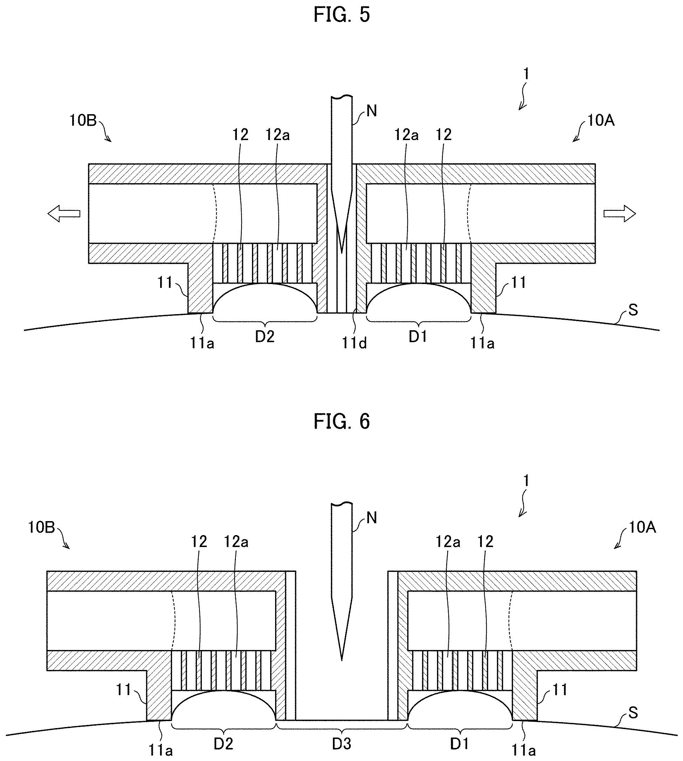

The reduction in pressure inside the frame 11 of the first fixing part 10A and in pressure inside the frame 11 of the second fixing part 10B causes a region (region D1 illustrated in Fig. 5) of the skin S of the living body inside the first end 11a of the frame 11 of the first fixing part 10A to be sucked toward the inner part of the frame 11 (see Fig. 5). This brings the skin S in the region D1 into a state of being attached to (in other words, a state of being suction-attracted to) the first fixing part 10A (more specifically, the inner part of the frame 11). Similarly, the skin S in a region (region D2 illustrated in Fig. 5) of the skin S of the living body inside the first end 11a of the frame 11 of the second fixing part 10B is brought into a state of being attached to (in other words, a state of being suction-attracted to) the second fixing part 10B (more specifically, the inner part of the frame 11).

Subsequently, in the above states, the first fixing part 10A and the second fixing part 10B are moved in directions (directions indicated by arrows in Fig. 5) in which the first fixing part 10A and the second fixing part 10B are away from each other (see Fig. 6). This causes a region (region D3 illustrated in Fig. 6) of the skin S of the living body between the region D1 and the region D2 to be pulled, so that the region D3 of the skin S is tensioned (see Fig. 6). Note that the first fixing part 10A and the second fixing part 10B can be moved manually by an operator or with use of a machine such as an actuator. The actuator can be, for example, an electromagnetic actuator, a piezoelectric actuator, a pneumatic actuator, or the like.

Next, the syringe is moved toward the skin S so that the needle N is inserted into the skin S of the living body. As described earlier, before the first fixing part 10A and the second fixing part 10B are moved, the needle N is located between the groove 11d formed in the frame 11 of the first fixing part 10A and the groove 11d formed in the frame 11 of the second fixing part 10B. This causes a place where the needle N is inserted into the skin S to be the region D3.

Skin of a living body is ordinarily depressed through the application of pressure by a needle during insertion of the needle into the skin. The depression in the skin stimulates a pain sensory nerve of the living body and thus causes pain to the living body. In contrast, according to the puncture method of Embodiment 1, the region D3 into which the needle N is inserted is tensioned as described earlier. This makes it possible to prevent the skin S from being depressed during the insertion of the needle N into the skin S. As a result, it is possible to insert the needle N into the skin S without stimulating a pain sensory nerve of the living body (in other words, without causing any pain to the living body).

Finally, by pulling up a plunger (not illustrated) stored inside the syringe, blood of the living body is sucked through the needle N so that the blood of the living body is collected.

As described earlier, the puncture assisting tool 1 of Embodiment 1 includes: the first fixing part 10A that is fixed to the region D1 serving as a first region of the skin S; and the second fixing part 10B that is fixed to the region D2 serving as a second region of the skin S. The first fixing part 10A is movable relative to the second fixing part 10B so as to pull the skin S in the region D3 between the first fixing part 10A and the second fixing part 10B.

According to the configuration, the skin in the region D3 can be tensioned in a case where the first fixing part 10A that is fixed to the region D1 of the skin S is moved relative to the second fixing part 10B that is fixed to the region D2 of the skin S. This makes it possible to prevent the skin S from being depressed during the insertion of the needle N into the skin S. As a result, it is possible to insert the needle N into the skin S without stimulating a pain sensory nerve of the living body.

The puncture assisting tool 1 includes the support 12 for supporting the skin S that has been sucked into the inner part of the frame 11. The support 12 is provided so as to be closer to the skin of the living body (an upper side in Fig. 3) than the air discharge part 13 in the axial direction (vertical direction in Fig. 3) of the cylindrical shape of the frame 11 (see Fig. 3). According to the configuration, the skin S that has been sucked into the inner part of the frame 11 is supported by the support 12 as illustrated in Fig. 5. This makes it possible to prevent the air discharge part 13 from being covered by the skin S. That is, it is possible to prevent air intake by the pump 20A or the pump 20B from being hampered. It is therefore possible to continuously suction-attract the skin S to the puncture assisting tool 1. Note that a puncture assisting tool of an aspect of the present invention can be configured to include no support 12.

According to the puncture assisting tool 1, the groove 11d for positioning the needle N in a state in which the first fixing part 10A and the second fixing part 10B are in contact with each other is formed in the frame 11 of each of the first fixing part 10A and the second fixing part 10B. This allows the place where the needle N is inserted into the skin S to be the region D3 that is tensioned.

Note that instead of the support 12, which is made of the flat plate, a plurality of columnar supports can be formed inside the frame 11 (on the second end 11b). The plurality of columnar supports support the skin S and can take in air from a space between the respective plurality of columnar supports.

### Embodiment 2

The following description will discuss another embodiment of the present invention. Note that for convenience, members having functions identical to those of the respective members described in Embodiment 1 are given respective identical reference numerals, and a description of those members is omitted.

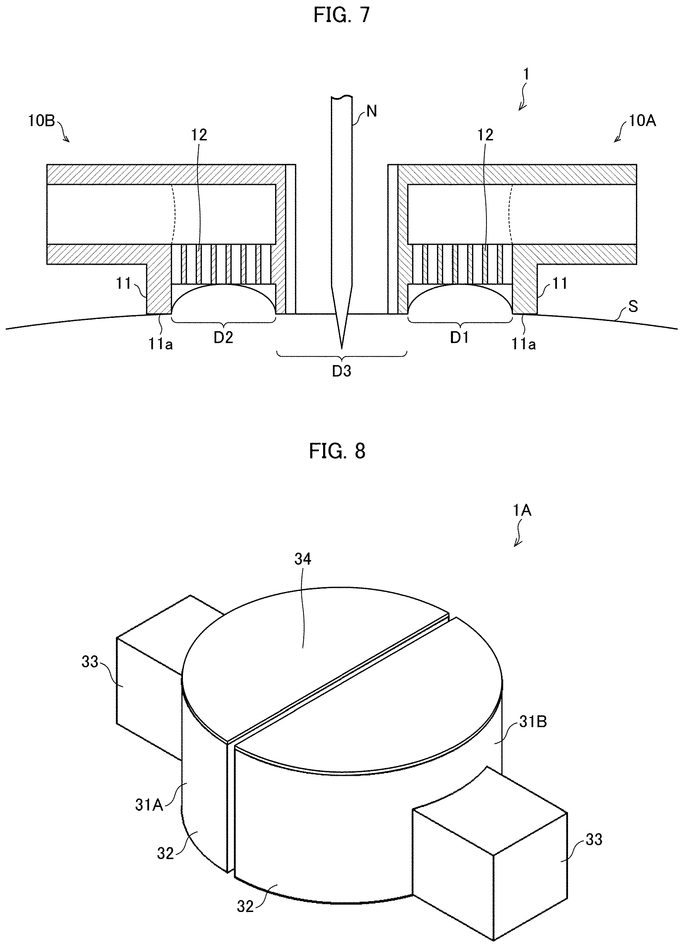

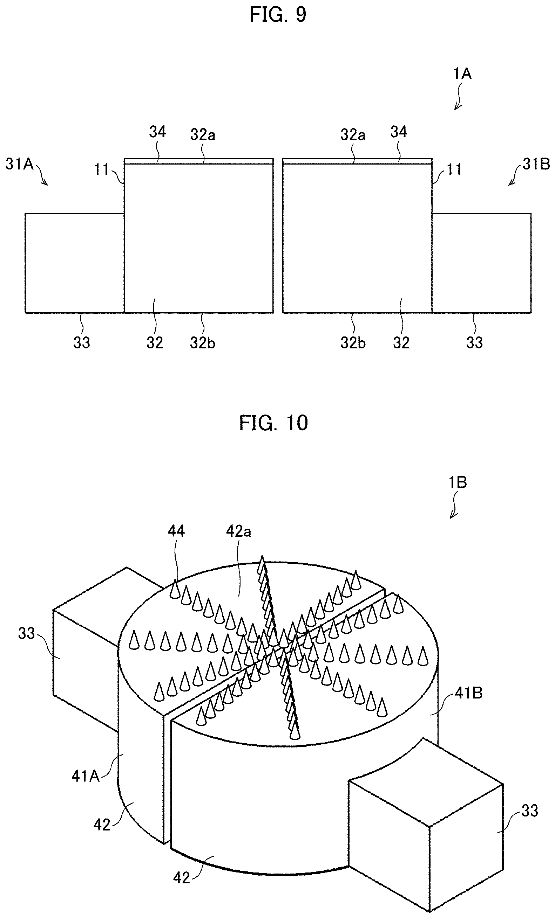

Fig. 8 is a perspective view of a puncture assisting tool 1A of Embodiment 2. Fig. 9 is a side view of the puncture assisting tool 1A. As illustrated in Figs. 8 and 9, the puncture assisting tool 1A includes a first fixing part 31A and a second fixing part 31B.

The first fixing part 31A and the second fixing part 31B have shapes that are symmetrical in the transverse direction in Fig. 9. Thus, only the first fixing part 31A will be described herein. The first fixing part 31A includes a semicylindrical member 32 and a gripping part 33.

The semicylindrical member 32 has a semicylindrical shape obtained by cutting a cylinder along a plane containing a central axis. The semicylindrical member 32 includes: a first end 32a, which is one end of a semicylinder and is an end on a side that contacts the skin of a living body; and a second end 32b, which is another end of the semicylinder. The first end 32a and the second end 32b are each made of a semicircular flat plate. An adhesive 34 (e.g., mucin or the like) is affixed to the first end 32a of the semicylindrical member 32.

The following description will discuss an example of a puncture method in which the puncture assisting tool 1A is used. According to the puncture method in which the puncture assisting tool 1A is used, first, the first end 32a of the semicylindrical member 32 of the first fixing part 31A and the first end 32a of the semicylindrical member 32 of the second fixing part 31B are brought into contact with skin S of the living body. This causes the adhesive 34 affixed to the first end 32a to stick to the skin S.

Subsequently, the gripping part 33 that is provided on a side surface of a frame 11 is gripped so that the first fixing part 31A and the second fixing part 31B are moved in directions in which the first fixing part 31A and the second fixing part 31B are away from each other. This causes a region of the skin S of the living body between regions in which the skin S has stuck to the adhesive 34 to be pulled, so that the region is tensioned.

Next, a needle N placed at a tip of a syringe (not illustrated) is inserted into the tensioned region of the skin S of the living body. The region into which the needle N is inserted is tensioned also in Embodiment 2. This makes it possible to prevent the skin S from being depressed during the insertion of the needle N into the skin S. As a result, it is possible to insert the needle N into the skin S without stimulating a pain sensory nerve of the living body.

Finally, by pulling up a plunger (not illustrated) stored inside the syringe, blood of the living body is sucked through the needle N so that the blood of the living body is collected.

In Embodiment 2, the adhesive 34 is used to cause the skin S to stick to the puncture assisting tool 1A. Note, however, that another method (e.g., an adhesive agent or the like) can be alternatively used to cause the skin S to adhere to the puncture assisting tool 1A.

### Embodiment 3

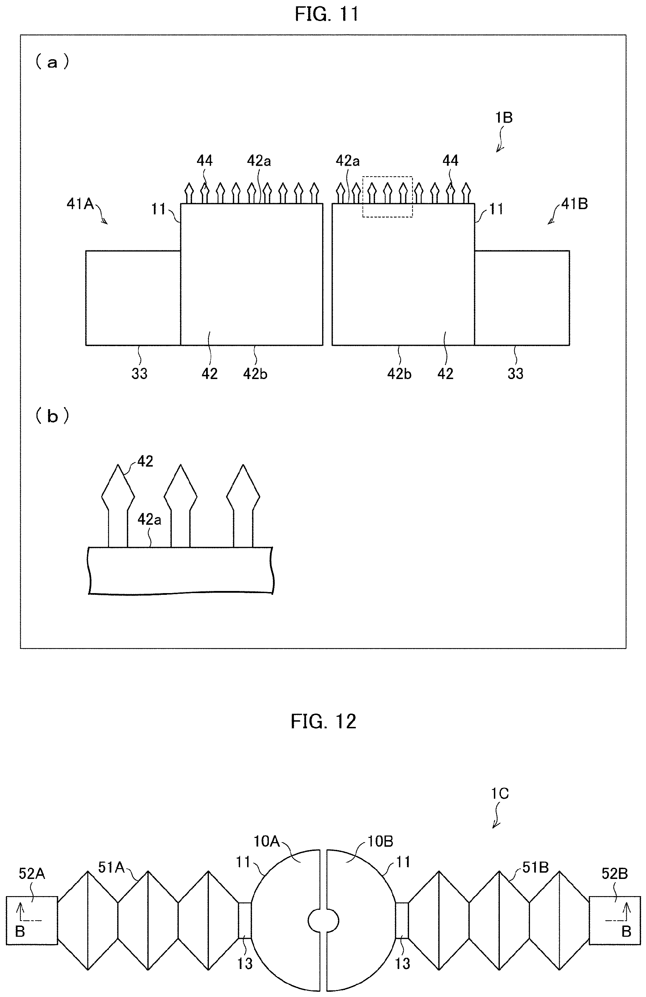

Fig. 10 is a perspective view of a puncture assisting tool 1B of Embodiment 3. (a) of Fig. 11 is a side view of the puncture assisting tool 1B. (b) of Fig. 11 is an enlarged view of a region surrounded with a frame in (a) of Fig. 11. As illustrated in Figs. 10 and 11, the puncture assisting tool 1B includes a first fixing part 41A and a second fixing part 41B.

The first fixing part 41A and the second fixing part 41B have shapes that are symmetrical in the transverse direction in Fig. 11. Thus, only the first fixing part 41A will be described herein. The first fixing part 41A includes a semicylindrical member 42 and a gripping part 33.

The semicylindrical member 42 has a semicylindrical shape obtained by cutting a cylinder along a plane containing a central axis. The semicylindrical member 42 includes: a first end 42a, which is one end of a semicylinder and is an end on a side that contacts the skin of a living body; and a second end 42b, which is another end of the semicylinder. The first end 42a and the second end 42b are each made of a semicircular flat plate. The first end 42a of the semicylindrical member 42 has a plurality of spikes 44 formed therein so as to protrude perpendicularly to the first end 42a. As illustrated in (b) of Fig. 11, the spikes 44 have a thorn shape and are provided with barbs. More specifically, the spikes 44 have a diamond-shaped cross section.

The following description will discuss an example of a puncture method in which the puncture assisting tool 1B is used. According to the puncture method in which the puncture assisting tool 1B is used, first, the first end 42a of the semicylindrical member 42 of the first fixing part 41A and the first end 42a of the semicylindrical member 42 of the second fixing part 41B are brought into contact with skin S of the living body. This causes the spikes 44 formed in the first end 42a to be caught by the skin S. Note that the spikes 44 have a length for which the spikes 44 are inserted into only a shallow part of the skin S (specifically, a stratum corneum and the epidermis). With the configuration, the living body does not feel any pain even if the spikes 44 are inserted into the skin S.

Subsequently, the gripping part 33 that is provided on a side surface of a frame 11 is gripped so that the first fixing part 41A and the second fixing part 41B are moved in directions in which the first fixing part 41A and the second fixing part 41B are away from each other. This causes a region of the skin S of the living body between regions in which the spikes 44 are inserted into the skin S to be pulled, so that the region is tensioned.

Note that the spikes 44 have a diamond-shaped cross section as described earlier. With the configuration, the spikes 44 (in other words, the first fixing part 41A and the second fixing part 41B) can be easily removed from the skin S of the living body when the spikes 44 are moved in a direction perpendicular to the first end 42a, and the spikes 44 cannot be removed from the skin S of the living body when the first fixing part 41A and the second fixing part 41B are moved in the directions in which the first fixing part 41A and the second fixing part 41B are away from each other.

Next, a needle N placed at a tip of a syringe (not illustrated) is inserted into the tensioned region of the skin S of the living body. The region into which the needle N is inserted is tensioned also in Embodiment 3. This makes it possible to prevent the skin S from being depressed during the insertion of the needle N into the skin S. As a result, it is possible to insert the needle N into the skin S without stimulating a pain sensory nerve of the living body.

### Embodiment 4

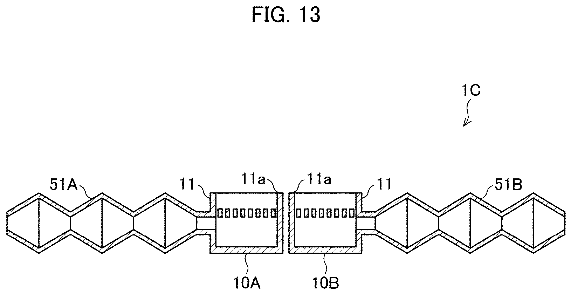

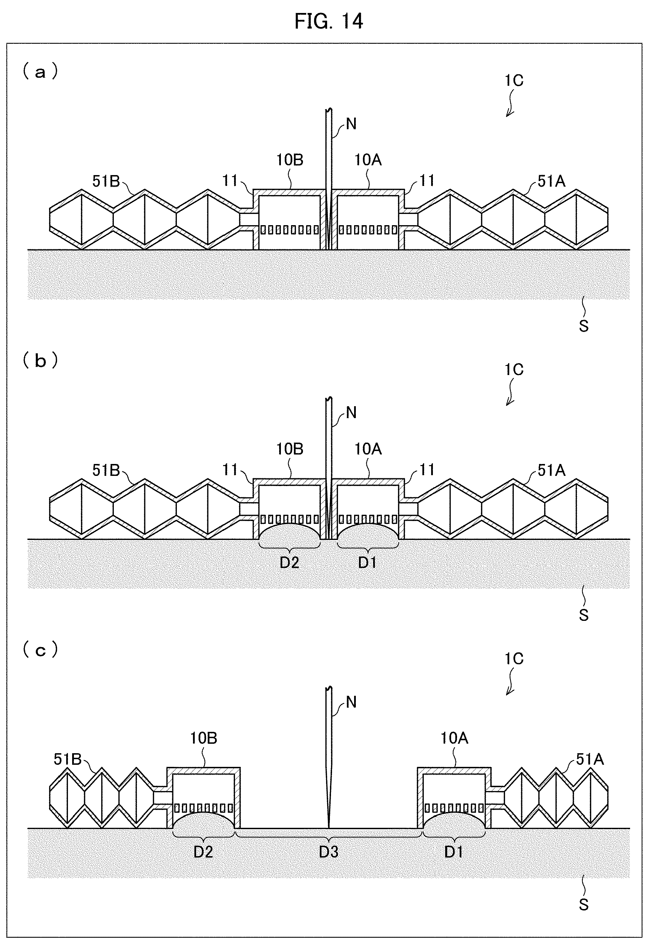

Fig. 12 is a perspective view of a puncture assisting tool 1C of Embodiment 4. Fig. 13 is a cross-sectional view taken along the line B-B illustrated in Fig. 12. Note that for simplification, a pump 52A and a pump 52B (each described later) are not illustrated in Figs. 13 and 14.

As illustrated in Figs. 12 and 13, the puncture assisting tool 1C includes a first fixing part 10A, a second fixing part 10B, an air flow part 51A, an air flow part 51B, the pump 52A, and the pump 52B.

The air flow part 51A has a bellows structure that contracts in response to pressure reduction. The air flow part 51A has one end that is connected via the air discharge part 13 to a space inside a frame 11 of the first fixing part 10A. The air flow part 51A has another end that is connected to the pump 52A.

Similarly, the air flow part 51B has a bellows structure that contracts in response to pressure reduction. The air flow part 51B has one end that is connected via the air discharge part 13 to a space inside the frame 11 of the second fixing part 10B. The air flow part 51B has another end that is connected to the pump 52B.

The following description will discuss an example of a puncture method in which the puncture assisting tool 1C is used. (a) to (c) of Fig. 14 are views each for describing an example of the puncture method in which the puncture assisting tool 1C is used.

According to the puncture method in which the puncture assisting tool 1C is used, first, the first fixing part 10A and the second fixing part 10B are brought into contact with skin S of a living body (see (a) of Fig. 14). Next, the pump 52A and the pump 52B are driven so that pressure inside the frame 11 of the first fixing part 10A and pressure inside the frame 11 of the second fixing part 10B are reduced.

The reduction in pressure inside the frame 11 of the first fixing part 10A and in pressure inside the frame 11 of the second fixing part 10B causes the skin S in a region D1 and the skin S in a region D2 to be attached to the first fixing part 10A and the second fixing part 10B, respectively (see (b) of Fig. 14).

Subsequently, the pump 52A and the pump 52B are further driven so that pressure inside the air flow part 51A and pressure inside the air flow part 51B are also reduced. This causes contraction of the respective bellows structures of the air flow part 51A and the air flow part 51B. This causes the first fixing part 10A and the second fixing part 10B to be moved in directions in which the first fixing part 10A and the second fixing part 10B are away from each other (see (c) of Fig. 14). This causes a region (region D3 illustrated in (c) of Fig. 14) of the skin S of the living body between the region D1 and the region D2 to be tensioned.

Next, a needle N placed at a tip of a syringe (not illustrated) is inserted into the tensioned region D3 of the skin S of the living body. The region D3 into which the needle N is inserted is tensioned also in Embodiment 4. This makes it possible to prevent the skin S from being depressed during the insertion of the needle N into the skin S. As a result, it is possible to insert the needle N into the skin S without stimulating a pain sensory nerve of the living body.

### Embodiment 5

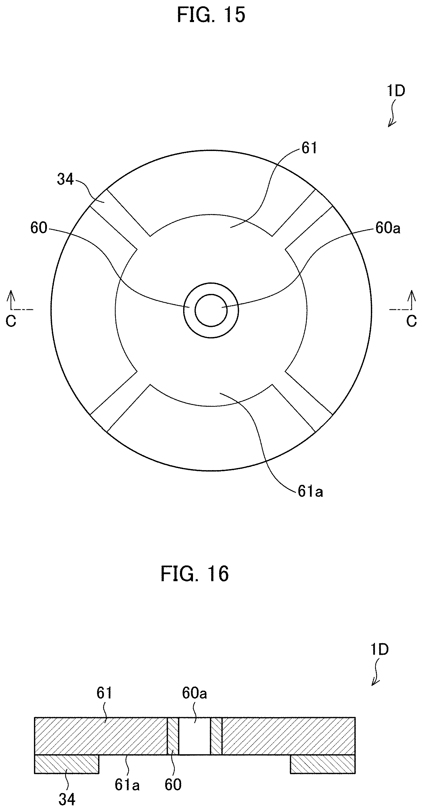

Fig. 15 is a top view of a puncture assisting tool 1D of Embodiment 5. Fig. 16 is a cross-sectional view taken along the line C-C illustrated in Fig. 15. As illustrated in Figs. 15 and 16, the puncture assisting tool 1D includes a guide part 60 and an expandable part 61.

The guide part 60 is provided at a center of the puncture assisting tool 1D. The guide part 60 has a cylindrical shape and has a through-hole 60a which is formed so as to extend in an axial direction (vertical direction in Fig. 16) of the cylindrical shape and through which a needle N passes. The guide part 60 is made of a rigid material. The guide part 60 is formed such that an inner diameter of the guide part 60 (in other words, a diameter of the through-hole 60a) is slightly larger than an outer diameter of the needle N. For example, the guide part 60 can be formed such that the inner diameter of the guide part 60 is 1.05 to 1.2 times larger than the outer diameter of the needle N.

The expandable part 61 is made of a material (e.g., a superabsorbent polymer or the like) which expands by addition thereto of water. The expandable part 61 is placed so as to surround a side surface of the guide part 60, and has a cylindrical shape. The expandable part 61 includes a first end 61a that faces skin S of a living body when the expandable part 61 is placed in the living body. As illustrated in Figs. 15 and 16, an adhesive 34 is affixed to a part of a region outside the first end 61a of the expandable part 61.

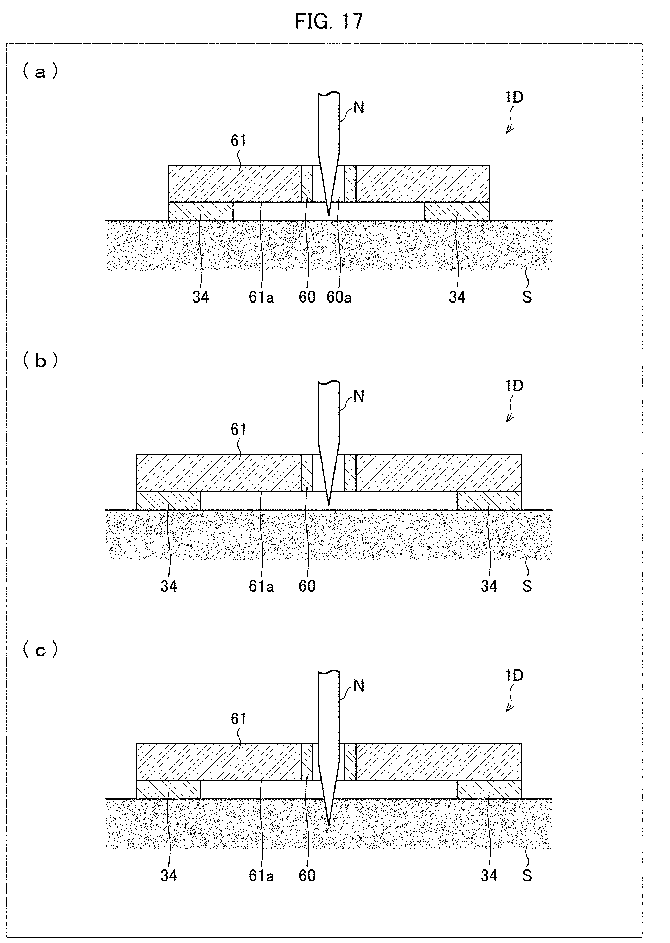

The following description will discuss an example of a puncture method in which the puncture assisting tool 1D is used. (a) to (c) of Fig. 17 are views each for describing an example of the puncture method in which the puncture assisting tool 1D is used.

According to the puncture method in which the puncture assisting tool 1D is used, first, the first end 61a side of the expandable part 61 is brought into contact with the skin S of the living body (see (a) of Fig. 17). This causes the adhesive 34 affixed to the first end 61a to stick to the skin S.

Next, the expandable part 61 is caused to absorb water. This causes the expandable part 61 to expand as illustrated in (b) of Fig. 17. In this case, since the guide part 60 is rigid, the expandable part 61 expands outward about a central axis of the guide part 60. This causes a region of the skin S of the living body between regions in which the skin S has stuck to the adhesive 34 to be pulled outward, so that the region is tensioned.

Next, a needle N placed at a tip of a syringe (not illustrated) is inserted into the tensioned region of the skin S of the living body. The region into which the needle N is inserted is tensioned also in Embodiment 5. This makes it possible to prevent the skin S from being depressed during the insertion of the needle N into the skin S. As a result, it is possible to insert the needle N into the skin S without stimulating a pain sensory nerve of the living body.

Finally, by pulling up a plunger (not illustrated) stored inside the syringe, blood of the living body is sucked through the needle N so that the blood of the living body is collected.

Furthermore, the puncture assisting tool 1D of Embodiment 5 includes the guide part 60 that is provided with the through-hole 60a through which the needle N passes and that guides movement of the needle N. With the configuration, since the needle N is guided by the guide part 60 during the insertion, it is possible to prevent buckling of the needle N. In other words, it is possible to use the needle N having a small outer diameter. As a result, it is possible to prevent stimulation of pain spots of the living body.

Embodiment 5 is configured such that the expandable part has a cylindrical shape. Note, however, that the present invention is not limited to the configuration. In an aspect of the present invention, the expandable part 61 can be rectangular when viewed in the direction of the central axis of the guide part 60.

### Embodiment 6

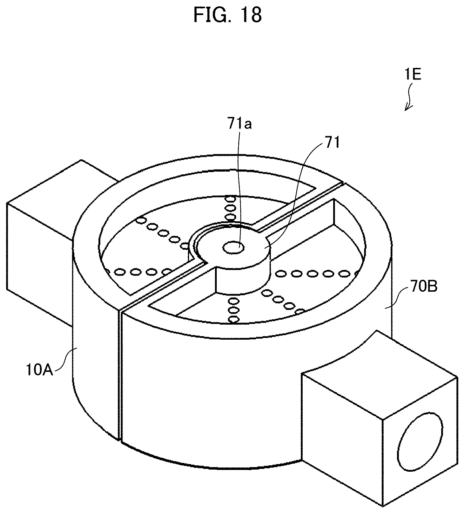

Fig. 18 is a perspective view of a puncture assisting tool 1E of Embodiment 6. As illustrated in Fig. 18, the puncture assisting tool 1D includes a second fixing part 70B instead of the second fixing part 10B of the puncture assisting tool 1 of Embodiment 1.

A guide part 71 is formed, at a center of a surface of the second fixing part 70B which surface faces a first fixing part 10A, so as to extend in a direction (vertical direction in Fig. 18) in which a needle N moves. The guide part 71 has a cylindrical shape and is provided with a through-hole 71a through which the needle N passes. The second fixing part 70B is similar in configuration to the second fixing part 10B of Embodiment 1, except that the second fixing part 70B has the guide part 71 formed therein.

With the configuration, the puncture assisting tool 1E allows skin in a region between a first region and a second region of skin S to be tensioned in a case where the first fixing part 10A that is fixed to the first region is moved relative to the second fixing part 70B that is fixed to the second region. This makes it possible to prevent the skin S from being depressed during the insertion of the needle N into the skin S. As a result, it is possible to insert the needle N into the skin S without stimulating a pain sensory nerve of the living body.

Furthermore, according to the puncture assisting tool 1E, since the needle N is guided by the guide part 71 during the insertion, it is possible to prevent buckling of the needle N. In other words, it is possible to use the needle N having a small outer diameter. As a result, it is possible to prevent stimulation of pain spots of the living body.

### Embodiment 7

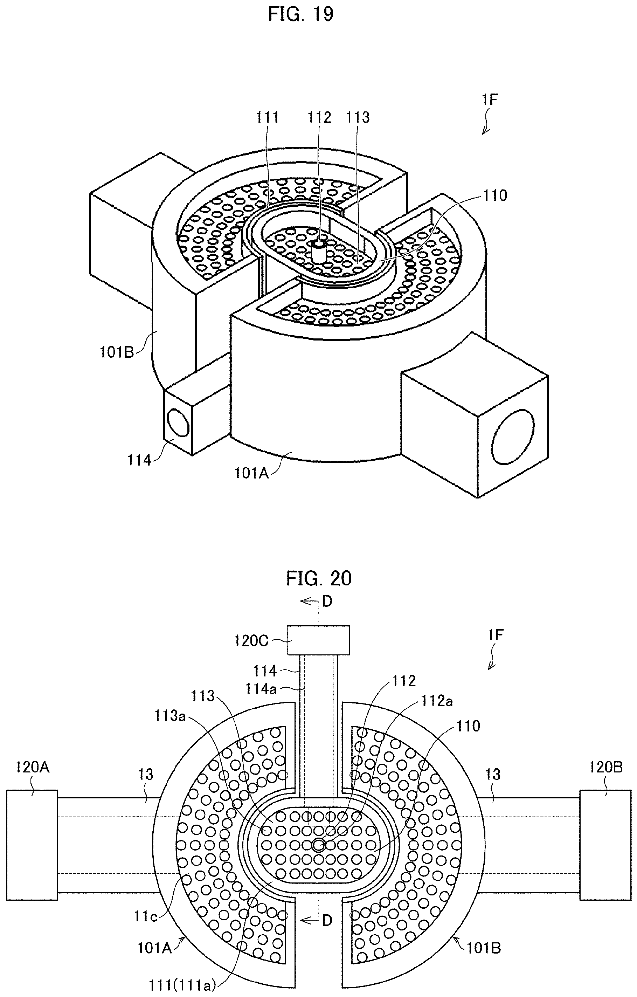

Fig. 19 is a perspective view of a puncture assisting tool 1F of Embodiment 7. Fig. 20 illustrates the puncture assisting tool 1F as viewed from an upward direction in Fig. 19.

As illustrated in Figs. 19 and 20, the puncture assisting tool 1F includes a first fixing part 101A, a second fixing part 101B, an attachment part 110, a pump 120A, a pump 120B, and a pump 120C. Note that in Fig. 19, the pumps 120A to 120C are not illustrated.

A description of the first fixing part 101A and the second fixing part 101B is omitted here. This is because the first fixing part 101A and the second fixing part 101B are similar in configuration to the first fixing part 10A and the second fixing part 101B, respectively, of Embodiment 1. As illustrated in Fig. 20, an air discharge part 13 provided to a frame 11 of the first fixing part 101A is connected to the pump 120A, and the air discharge part 13 provided to the frame 11 of the second fixing part 101B is connected to the pump 120B.

The attachment part 110 is located between the first fixing part 101A and the second fixing part 101B. The attachment part 110 includes a frame 111, a guide part 112, a support 113, and an air discharge part 114.

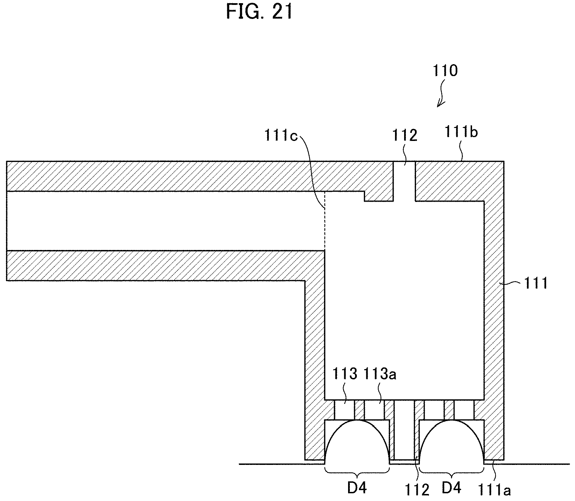

The frame 111 has a tubular shape. The frame 11 includes: a first end 111a, which is one end of a tube and is an end on a side that contacts the skin of a living body; and a second end 111b, which is another end of the tube (see Fig. 21). The first end 111a is shaped to have an opening on the inside, and the second end 111b is made of a flat plate. Thus, the frame 111 has a shape in which a face thereof on the side that contacts the skin of a living body is open.

Further, the frame 111 includes an opening 111c formed on a side surface of a cylinder and in a region where an air discharge part 114 (described later) is installed (see Fig. 21).

The guide part 112 is a member for guiding movement of a needle N. The guide part 112 has a cylindrical shape. The cylindrical shape is coaxial with the cylindrical shape of the frame 11. The guide part 112 has a through-hole 112a which is formed so as to extend in an axial direction (vertical direction in Fig. 19) of the tube and through which the needle N passes.

The support 113, in an inner part of the frame 111, is formed between the first end 111a and the second end 111b in the axial direction (vertical direction in Fig. 19) of the cylindrical shape of the frame 111. The support 113 is made of a flat plate. The support 113 has a plurality of air intake holes 113a formed therein.

The air discharge part 114 is provided on the side surface of the frame 111. The air discharge part 114 has a cylindrical hole 114a formed therein. As illustrated in Fig. 20, the hole 114a has (i) one end that is connected to an opening 111c provided in the frame 111 and (ii) the other end that is connected to the pump 120C.

The following description will discuss an example of a puncture method in which the puncture assisting tool 1F is used. First, the puncture assisting tool 1F is brought into contact with skin S of a living body. Next, the pump 120A and the pump 120B are driven. This reduces pressure inside the first fixing part 101A and pressure inside the second fixing part 101B.

The reduction in pressure inside the frame 11 of the first fixing part 101A results in a state in which a region (first region) of the skin S of the living body inside a first end 11a of the frame 11 of the first fixing part 101A is attached to the frame 11 of the first fixing part 101A. Similarly, the reduction in pressure inside the frame 11 of the second fixing part 101B results in a state in which a region (second region) of the skin S of the living body inside the first end 11a of the frame 11 of the second fixing part 101B is attached to the frame 11 of the second fixing part 101B.

Subsequently, in the above states, the first fixing part 101A and the second fixing part 101B are moved in directions in which the first fixing part 101A and the second fixing part 101B are away from each other. This causes a region (third region) of the skin S of the living body between the first region and the second region to be pulled, so that the third region is tensioned.

Then, the pump 120C is driven. This sucks air inside the frame 111 of the attachment part 110. This reduces pressure inside the frame 111 of the attachment part 110. Note that since the support 113 of the frame 111 of the attachment part 110 has the plurality of air intake holes 113a formed therein, it is possible to reduce the pressure in the whole inner part of the frame 111.

Fig. 21 is a cross-sectional view taken along the line D-D of Fig. 20 and illustrating a state in which pressure inside the frame 111 of the attachment part 110 is reduced during puncture in which the puncture assisting tool 1F is used. The reduction in pressure inside the attachment part 110 of the frame 111 results in a state in which a region (region D4 illustrated in Fig. 21) of the skin S of the living body inside the first end 111a of the frame 111 of the attachment part 110 is attached to the inside of the frame 111 as illustrated in Fig. 21.

Next, a syringe (not illustrated) is moved toward the skin S so that the needle N is inserted into the skin S of the living body. A region into which the needle N is inserted is tensioned. This makes it possible to prevent the skin S from being depressed during the insertion of the needle N into the skin S. As a result, it is possible to insert the needle N into the skin S without stimulating a pain sensory nerve of the living body.

Furthermore, according to Embodiment 7, the skin S is attached to the inner part of the frame 111 of the attachment part 110. With the configuration, the region into which the needle N is inserted is further tensioned as compared with Embodiment 1. This makes it possible to prevent the skin S from being depressed during the insertion of the needle N into the skin S.

Moreover, according to the puncture assisting tool 1F, the needle N is guided, during the puncture, by the guide part 112 provided in the attachment part 110. This makes it possible to prevent buckling of the needle N. In other words, it is possible to use the needle N having a small outer diameter. As a result, it is possible to prevent stimulation of pain spots of the living body.

Further, according to the puncture assisting tool 1F, the guide part 112 is located inside a region of the attachment part 110 to which region the skin S is attached. This allows a place where the needle N is inserted into the skin S of the living body to be a region of the skin S inside the attachment part 110 region.

In Embodiment 7, the pumps 120A to 120C are used as (i) a suction device for sucking air inside the first fixing part 101A, (ii) a suction device for sucking air inside the second fixing part 101B, and (iii) a suction device for sucking air inside the attachment part 110, respectively. However, the present invention is not limited to this. In an aspect of the present invention, a syringe can be used as a suction device.

In addition, a mechanism for use in a puncture assisting tool of the present invention can be used for endoscope treatment. Specifically, by using the first fixing part and the second fixing part of the present invention to tension skin around a tumor developed in, for example, an organ of a living body, it is possible to easily cut the tumor, tattoo the tumor, and the like.

### Reference Signs List

1, 1A-1F Puncture assisting tool
10A, 31A, 41A, 101A First fixing part
10B, 31B, 41B, 70B, 101B, 102B Second fixing part
11d Groove
12 Support
20A, 120A Pump (suction device)
44 Spike
51A, 51B Air flow part
60, 71, 112 Guide part
60a, 71a, 112a Through-hole
110 Attachment part
D1 Region (first region)
D2 Region (second region)
N Needle
S Skin

## Claims

1. A puncture assisting tool (1) comprising:
a first fixing part (10A) that is fixed to a first region of skin; and
a second fixing part (10B) that is fixed to a second region of the skin;
**characterized in that**:
the first fixing part (10A) is movable relative to the second fixing part (10B) so as to pull the skin between the first fixing part (10A) and the second fixing part (10B),
the first fixing part (10A) suction-attracts the skin, and
the first fixing part (10A) has a support (12) that supports the skin which has been sucked into the first fixing part (10A),
the second fixing part (10B) suction-attracts the skin, and
the second fixing part (10B) has a support (12) that supports the skin which has been sucked into the second fixing part (10B).

2. The puncture assisting tool (1) as set forth in claim 1, wherein the first fixing part (10A) is provided with a groove (11d) for positioning a needle in a state in which the first fixing part (10A) and the second fixing part (10B) are in contact with each other.

3. A puncture assisting tool as (1F) set forth in claims 1 or 2, further comprising a guide part (112) that is provided with a through-hole (112a) through which a needle passes and that guides movement of the needle.

4. A puncture assisting tool (1F) as set forth in claim 3, further comprising an attachment part (110) that is attached to the skin,
the guide part (112) being located inside a region of the attachment part (110) in which region the attachment part is attached to the skin.

5. A puncture assisting tool (1C) as set forth in any one of claims 1 to 4, further comprising an air flow part (51A) that is connected to a space inside the first fixing part (10A) and that has a bellows structure which contracts in response to pressure reduction.

6. A puncture assisting tool (1) as set forth in any one of claims 1 to 5, further comprising a suction device (20A) that sucks air inside the first fixing part (10A).

## Patentansprüche

1. Einstichunterstützendes Werkzeug (1), umfassend:
ein erstes Befestigungsteil (10A), das an einem ersten Hautbereich befestigt ist; und
ein zweites Befestigungsteil (10B), das an einem zweiten Hautbereich befestigt ist;
**gekennzeichnet durch**:
das erste Befestigungsteil (10A) ist relativ zum zweiten Befestigungsteil (10B) beweglich, um die Haut zwischen das erste Befestigungsteil (10A) und das zweite Befestigungsteil (10B) zu ziehen,
das erste Befestigungsteil (10A) saugt die Haut an, und
das erste Befestigungsteil (10A) weist eine Stütze (12) auf, die die Haut stützt, die in das erste Befestigungsteil (10A) gesaugt worden ist,
das zweite Befestigungsteil (10B) saugt die Haut an, und
das zweite Befestigungsteil (10B) weist eine Stütze (12) auf, die die Haut stützt, die in das zweite Befestigungsteil (10B) gesaugt worden ist.

2. Einstichunterstützendes Werkzeug (1) nach Anspruch 1, wobei das erste Befestigungsteil (10A) mit einer Nut (11d) zum Positionieren einer Nadel in einem Zustand versehen ist, in dem das erste Befestigungsteil (10A) und das zweite Befestigungsteil (10B) miteinander in Kontakt sind.

3. Einstichunterstützendes Werkzeug (1F) nach Anspruch 1 oder 2, das ferner ein Führungsteil (112) umfasst, das mit einem Durchgangsloch (112a) versehen ist, durch das eine Nadel verläuft und das die Bewegung der Nadel führt.

4. Einstichunterstützendes Werkzeug (1F) nach Anspruch 3, ferner umfassend ein Befestigungsteil (110), das an der Haut befestigt ist,
wobei das Führungsteil (112) innerhalb eines Bereichs des Befestigungsteils (110) angeordnet ist, in dem das Befestigungsteil an der Haut befestigt ist.

5. Einstichunterstützendes Werkzeug (1C) nach einem der Ansprüche 1 bis 4, ferner umfassend ein Luftströmungsteil (51A), das mit einem Raum innerhalb des ersten Befestigungsteils (10A) verbunden ist und das eine Balgstruktur aufweist, die sich als Reaktion auf eine Druckreduzierung zusammenzieht.

6. Einstichunterstützendes Werkzeug (1) nach einem der Ansprüche 1 bis 5, ferner mit einer Saugvorrichtung (20A), die Luft in das Innere des ersten Befestigungsteils (10A) saugt.

## Revendications

1. Outil d'assistance à la piqûre (1) comprenant :
une première partie de fixation (10A) fixée à une première région de la peau ; et
une deuxième partie de fixation (10B) fixée à une deuxième région de la peau ;
**caractérisé en ce que** :
la première partie de fixation (10A) est mobile par rapport à la deuxième partie de fixation (10B) de manière à tirer la peau entre la première partie de fixation (10A) et la deuxième partie de fixation (10B),
la première partie de fixation (10A) attire la peau par aspiration, et
la première partie de fixation (10A) présente un support (12) qui soutient la peau qui a été aspirée dans la première partie de fixation (10A),
la deuxième partie de fixation (10B) attire la peau par aspiration, et
la deuxième partie de fixation (10B) présente un support (12) qui soutient la peau qui a été aspirée dans la deuxième partie de fixation (10B).

2. Outil d'assistance à la piqûre (1) selon la revendication 1, dans lequel la première partie de fixation (10A) est pourvue d'une rainure (11d) pour positionner une aiguille dans un état dans lequel la première partie de fixation (10A) et la seconde partie de fixation (10B) sont en contact l'une avec l'autre.

3. Outil d'assistance à la piqûre (1F) selon la revendication 1 ou 2, comprenant en outre une partie de guidage (112) pourvue d'un trou traversant (112a) à travers lequel passe une aiguille et lequel guide le mouvement de l'aiguille.

4. Outil d'assistance à la piqûre (1F) selon la revendication 3, comprenant en outre une partie d'attachement (110) qui est attachée à la peau,
la partie de guidage (112) étant située à l'intérieur d'une zone de la partie de fixation (110) dans laquelle la partie d'attachement est attachée à la peau.

5. Outil d'assistance à la piqûre (1C) selon l'une des revendications 1 à 4, comprenant en outre une partie d'écoulement d'air (51A) qui est reliée à un espace à l'intérieur de la première partie de fixation (10A) et qui présente une structure de soufflet qui se contracte en réponse à une réduction de la pression.

6. Outil d'assistance à la piqûre (1) selon l'une des revendications 1 à 5, comprenant en outre un dispositif d'aspiration (20A) qui aspire de l'air dans la première partie de fixation (10A).
